# DEMANDE DE BREVET EUROPEEN

(11) **EP 1 110 555 A1**
(43) Date de publication de la demande: **27.06.2001**
(21) Numéro de dépôt: 99204489.1
(22) Date de dépôt: 22.12.1999
(51) Int. Cl.: A61K 35/74, A61P 17/00

(54) **Agent anti-adhesion de la flore pathogene de la peau**

(71) Demandeur: SOCIETE DES PRODUITS NESTLE S.A., 1800 Vevey (CH)
(72) Inventeur: Baur, Markus, 1603 Aran (CH); Zink, Ralf, 1801 Le Mont Pelerin (CH); Auzanneau, Isabelle, 06650 Opio (FR); Buffard, Karine, 06250 Mougins-Le-Haut (FR)

(57) **Abrégé**

Utilisation d'un agent bactérien pour la préparation de compositions à usage cosmétique, pharmaceutique ou vétérinaire destinées à stabiliser et/ou réguler l'écosystème cutané des mammifères, ledit agent bactérien étant un extrait de bactérie ou une bactérie sélectionnée pour ses facultés d'adhésion vis-à-vis des cellules de la peau et ses propriétés anti-adhésives vis-à-vis des pathogènes du système cutané; et les compositions contenant un tel agent.

## Description

La présente invention a pour objet l'utilisation d'un agent bactérien sélectionné pour ses facultés d'anti-adhésion de pathogènes cutanés pour la préparation de compositions à usage cosmétique, pharmaceutique ou vétérinaire destinées à stabiliser et/ou réguler l'écosystème cutané des mammifères et les compositions contenant un tel agent.

### État de la technique

La prolifération de pathogènes tels que *Staphylococcus aureus, Streptococcus pyogenes* ou *Propionibacterium acnes*, ou de certaines levures, peuvent entraîner une dysrégulation du système cutané voire des désordres plus graves au niveau de la peau ou des muqueuses tels qu'éczema, candidoses, dermites, etc.

On connaît de nombreux moyens de traitement contre ces agents pathogènes. Les antibiotiques ou anti-bactériens chimiques sont les plus classiquement utilisés. Ce sont par exemple des compositions à base d'aldhéhydes et dérivés.

Ainsi, la demande de brevet publiée FR 2740039, décrit l'utilisation d'une substance choisie parmi les aldhéhydes et les composés bi-fonctionnels, de préférence le glutaraldéhyde, pour inhiber la fixation sur les kératinocytes et cornéocytes de souches de pathogènes tels que *Staphylococcus aureus.*

Aussi, l'hexachlorophene et ses dérivés, sont connus comme substances antibactériennes et sont plus particulièrement utilisés contre *Propionibacterium acnes.*

Ces traitements sont en général côuteux et nocifs aussi bien pour la santé que pour l'environnement.

On connaît maintenant des traitements alternatifs, non-toxiques, qui consistent à utiliser les propriétés anti-fongiques, bactéricides ou bactériostatiques de certaines souches de microorganismes.

Ainsi, la demande PCT WO 97/366603 met en évidence les propriétés antifongiques d' une souche de *Lactobacillus casei.*

D'autres agents bacteriens tels que les *Bacillus* peuvent aussi être utilisés sur la peau ou les muqueuses. En effet, dans la demande WO 98/47374, des souches de *Bacillus coagulans, Bacillus subtilis, Bacillus laterosporus* et *Bacillus laevolacticus* sont utilisées dans des compositions destinées à prévenir les infections bactériennes, virales ou fongiques de la peau ou des muqueuses.

L'invention se propose de trouver un nouvel agent bactérien, capable de contrôler et réguler l'écosystème cutané.

### Résumé de l'invention

A cet effet, la présente invention concerne l'utilisation d'un agent bactérien pour la préparation d'une composition à usage cosmétique, pharmaceutique ou vétérinaire, destinée à être administrées à l'homme ou à des animaux dans le but de prévenir ou traiter les désordres induits par les pathogènes du système cutané, ledit agent bactérien étant un extrait de bactérie lactique ou une bactérie lactique sélectionnée pour ses facultés d'adhésion vis-à-vis des cellules cutanées et de régulation de l'attachement de pathogènes cutanés, notamment par inhibition de leur adhésion.

L'agent bactérien peut être choisi parmi des souches de *Lactobacillus, Micrococcus* ou *Bifidobacterium*, et de préférence parmi les souches de *Lactobacillus johnsonii* CNCM I-1225, *Micrococcus varians* CNCM I-1586, *Micrococcus varians* CNCM I-1587 ou *Bifidobactérium lactis* ATCC 27536, par exemple.

La souche de bactérie peut être utilisée sous une forme viable, désactivée ou semi-active.

L'agent bactérien peut être utilisé sous forme d'une poudre lyophilisée, par exemple, qui peut comprendre environ 10E+08 à 10E+11 cfu/g.

La présente invention concerne également une composition à usage cosmétique, pharmaceutique ou vétérinaire, contenant au moins un agent bactérien capable de stabiliser et/ou de réguler la flore pathogène du système cutané, ledit agent bactérien étant un extrait de bactérie ou une bactérie sélectionnée pour ses facultés d'adhésion vis-à-vis des cellules de la peau et ses propriétés anti-adhésives vis-à-vis des pathogènes du système cutané.

Cette composition peut également avoir un usage en ophtalmologie ou pour une application nasale.

Cette composition peut être notamment sous forme de crème, lotion, pain dermatologique, shampooing ou poudre, par exemple.

### Description détaillée de l'invention

On regroupe sous la dénomination "système cutané", l'ensemble des cellules de la peau (i.e. kératinocytes) mais également les cornéocytes.

La présente invention propose un agent bactérien sélectionné pour sa faculté d'adhérence à des cellules cutanées, de stabilisation et de régulation de la flore bactérienne pathogène du système cutané, notamment par inhibition de l'adhésion de pathogènes tels que *Staphylococcus aureus, Streptococcus pyogenes* ou *Propionibacterium acnes*, par exemple.

Pour cela de nombreuses souches de bactéries ont été testées pour leurs propriétés d'attachement sur des kératinocytes humains (cf exemple 1).

Parmi diverses souches de bactéries ainsi testées, on a sélectionné des souches de Lactobacillus, de Micrococcus et de Bifidobacterium, et plus particulièrement une souche de *Lactobacillus johnsonii* (NCC 533), deux souches de *Micrococcus varians* (NCC 1482, NCC 1520) et une souche de *Bifidobacterium lactis* (ATCC 27536).

La souche de *Lactobacillus johnsonii* (NCC 533) et les souches de *Micrococcus varians* NCC 1482 et NCC 1520 ont fait l'objet d'un dépôt, selon le Traité de Budapest, à la Collection nationale de Cultures de Microorganismes (CNCM), Institut Pasteur, 28 rue du Docteur Roux, 75724 Paris Cedex 15, France, respectivement le 30 juin 1992 sous la référence CNCM I-1225 pour *Lactobacillus johnsonii* et 07 juin 1995 sous les références et CNCM I-1586 et CNCM I-1587 pour les *Micrococcus varians* NCC 1482 et NCC 1520.

La souche de *Bifidobacterium lactis* (ATCC27536), peut être obtenue chez Hansen (Chr. Hansen A/S, 10-12 Boege Alle, P.O. Box 407, DK-2970 Hoersholm, Danemark).

Des détails concernant la morphologie et les propriétés générales des souches sont données ci-après:

### Lactobacillus johnsonii CNCM I-1225

Morphologie
   - Microorganisme Gram-positif, non motile, ne formant pas de spores.
   - Bâtonnets isolés assez courts et trapus.
Métabolisme
   - Microorganisme microaérophile, avec métabolisme homofermentaire donnant lieu à la production d'acide lactique L (+) et D (-).
   - Autres caractéristiques : Catalase (-), production de CO2 (-), hydrolyse de l'arginine (-).
Fermentation des sucres :
   Amygdaline (+), arabinose (-), cellobiose (+), esculine (+), fructose (+), galactose (-), glucose (+), lactose (+), maltose (+/-), mannitol (-), mannose (+), melibiose (-), raffinose (+), ribose (-), salicine (+), sucrose (+), tréhalose (+).

### Micrococcus varians CNCM I-1586 (NCC 1482) et CNCM I-1587 (NCC 1520)

Morphologie
   - Microorganisme Gram-positif, sont immobiles de manière permanente
   - Forme sphérique, se présente sous forme de tétrades arrangées irrégulièrement.
Métabolisme
   - Microorganisme aérobe, Catalase (+)
   - Autres caractéristiques : couleur jaune sur milieu BHI. La température optimale de croissance desdites souches est de 25-37° C.
Fermentation des sucres
   fructose (+), glucose (+).

Les bactéries selon l'invention sont utilisées pour la préparation de compositions destinées au traitement prophylactique ou thérapeutique de désordres liés aux pathogènes du système cutané tels que *Staphylococcus aureus, Streptococcus pyogenes* ou *Propionibacterium acnes*, ou des levures, par exemple.

Ces désordres cutanés peuvent être notamment la dermite atopique (dans les phases de rémission comme traitement d'entretien), l'acné, les candidoses, la dermite séborrhéique, le pityriasis versicolor, l'impetigo ou les surinfections eczémateuses.

Les désordres du système cutané peuvent aussi être liés à des thérapeutiques avec des antibiotiques, des anti-mycosiques, au diabète (candidoses), à une pathologie des muqueuses (candidose vaginale), à de l'eczéma chronique (déséquilibre de l'homéostase), à des peaux sensibles (prématurés, enfants), des peaux grasses (lié à des dysrégulations hormonales qui peuvent favoriser la pousse de bactéries) ou à des états pelliculaires.

Les bactéries selon l'invention peuvent être utilisées sous leur forme vivante, semi-active ou sous une forme désactivée. On entend par bacterie sous une forme semi-active, une bactérie dont l'activité physiologique est réduite. Cette activité peut être mesurée par une phase exponentielle de croissance ou un temps de génération supérieur, un métabolisme ralenti ou une réponse physiologique incomplète à des modifications de l'environnement, par exemple. Dans certains cas extrêmes, le nombre des bactéries peut être diminué puisqu'elles ne peuvent plus résister au changement de l'environnement.

Les surnageants de culture des bactéries peuvent également être utilsés.

Selon un premier mode de réalisation de l'invention, l'agent bactérien peut être un extrait de bactérie ou une bactérie, ladite bactérie étant sous sa forme viable active. L'agent bactérien se présente alors de préférence sous forme d'une poudre lyophilisée, obtenue par exemple par le procédé décrit dans EP 818529. La poudre peut contenir de 10E+08 à 10E+11 cfu/g.

Selon un autre mode de réalisation, l'agent bactérien peut être un extrait de bactérie ou une bactérie sous une forme semi-active. La désactivation partielle des souches peut être réalisée de plusieurs manière, notamment par :
- freeze drying consistant en cycles de congélation dans l'azote liquide / décongélation à 37°C. On peut alors obtenir une réduction d'environ 1 log,
- action des UV (15 à 60 minutes à 254 nm, distance 20 cm) : réduction de 2 à 3 logs,
- action de la chaleur (70°C pendant 3 heures ) : réduction d'environ 3 à 4 logs, par exemple.

L'agent bactérien peut être alors utilisé sous forme de poudre contenant au moins 10E+6 cfu/g, et de préférence dans des compositions sèches telles que shampoings secs ou autres compositions poudreuses qui peuvent contenir jusqu'à 10% de l'extrait bactérien.

Enfin, l'agent bactérien peut être également un extrait de bactérie ou une bactérie sous une forme désactivée. La bactérie est de préférence inactivée par traitement thermique à environ 90°C pendant environ 2 heures. L'agent bactérien se présente sous forme d'une poudre lyophilisée contenant de 10E+08 à 10E+12 cfu/g. Il peut être utilisé jusqu'à 5% et de préférence de 0,05 à 3% dans des compositions liquides et jusqu'à 10% dans des compositions pulvérulentes.

La présente invention concerne également une composition à usage cosmétique, pharmaceutique ou vétérinaire contenant agent bactérien ayant les propriétés décrites ci-avant.

Pour préparer une telle composition, au moins une souche de bactérie sous forme viable, semi-active ou désactivée, est incorporée à un support pharmaceutiquement ou cosmétiquement acceptable en quantité variant en fonction de l'application recherchée. L'agent bactérien peut être présent jusqu'à environ 5% par rapport au poids total de la composition et jusqu'à 10% pour des compositions sous forme de poudre, et de préférence entre 0,5 à 2% .

Les compositions selon l'invention peuvent être administrées par voie topique ou occulaire.

Par voie topique, les compositions pharmaceutiques à base de composés selon l'invention sont de préférence destinées au traitement de la peau et des muqueuses et peuvent se présenter sous forme d'onguents, de crèmes, de laits, de pommades, de poudres, de tampons imbibés, de solutions, de gels, de sprays, de lotions ou de suspensions. Elles peuvent également se présenter sous forme de microsphères ou nanosphères ou vésicules lipidiques ou polymériques ou de patches polymériques et d'hydrogels permettant une libération contrôlée. Ces compositions par voie topique peuvent se présenter soit sous forme anhydre, soit sous forme aqueuse selon l'indication clinique.

Par voie oculaire, ce sont principalement des collyres.

La présente invention vise plus particulièrement une composition cosmétique contenant, dans un support cosmétiquement acceptable, au moins un agent bactérien tel que défini ci-dessus. La composition cosmétique peut contenir l'agent bactérien à raison d'au moins 0,001 % en poids par rapport au poids total de la composition, et de préférence de 0,05 à 3%.

Cette composition cosmétique est notamment destinée à l'hygiène corporelle et capillaire. Elle peut se présenter notamment sous forme d'une crème, d'un lait, d'une lotion, d'un gel, de microsphères ou nanosphères ou vésicules lipidiques ou polymériques, d'un savon ou d'un shampooing.

Dans les compositions selon l'invention, l'agent bactérien viable ou inactivé peut être combiné avec des rétinoïdes ou des corticostéroïdes, ou associé avec des anti-radicaux libres, avec des α-hydroxy ou α-céto acides ou leurs dérivés, ou encore avec des bloqueurs de canaux ioniques.

Les compositions pharmaceutiques et cosmétiques selon l'invention peuvent, en outre, contenir des additifs inertes ou même pharmacodynamiquement ou cosmétiquement actifs ou des combinaisons de ces additifs et notamment : des agents mouillants; des agents dépigmentants tels que l'hydroquinone, l'acide azelaïque, l'acide caféïque ou l'acide kojique; des émollients; des agents hydratants comme le glycérol, le PEG-400, la thiamorpholinone et ses dérivés ou l'urée; des agents antiséborrhéiques ou antiacnéiques, tels que la S-carboxyméthylcystéine, la S-benzyl-cystéamine, leurs sels et leurs dérivés, ou le péroxyde de benzoyle; des antibiotiques comme l'érythromycine et ses esters, la néomycine, la clindamycine et ses esters, les tétracyclines; des agents antifongiques tels que le kétoconazole ou les polyméthylène-4,5 isothiazolinones-3 ; des agents favorisant la repousse des cheveux, comme le Minoxidil (2,4-diamino-6-pipéridino-pyrimidine-3-oxyde) et ses dérivés, le Diazoxide (7-chloro 3-méthyl 1,2,4- benzothiadiazine 1,1-dioxyde) et le Phénytoïn (5,4-diphényl-imidazolidine 2,4-dione) ; des agents anti-inflammatoires non stéroïdiens; des caroténoïdes et, notamment le β-carotène; des agents anti-psoriatiques tels que l'anthraline et ses dérivés et enfin, les acides eicosa-5,8,11,14- tétraynoïque et eicosa-5,8,11-trynoïque, leurs esters et les amides.

La composition selon l'invention peut également contenir des agents conservateurs tels que les esters de l'acide parahydroxybenzoïque, les agents stabilisants, des agents régulateurs d'humidité, des agents régulateurs de pH, des agents modificateurs de pression osmotique, des agents émulsionnants, des filtres UV-A et UV-B, des anti-oxydants, tels que l'α-tocophérol, le butylhydroxyanisole ou le butylhydroxytoluène.

La présente invention vise enfin une composition à usage vétérinaire ou cosmétique pour animaux, contenant au moins un agent bactérien tel que défini ci-dessus. Une telle composition peut se présenter sous forme de shampooings secs ou liquides, poudres, mousses ou lotions par exemple. Elle peut contenir jusqu'à 10% de l'agent bactérien.

La composition selon l'invention est destinée en particulier au traitement thérapeutique ou prophylactique des peaux et/ou muqueuses saines, sensibles et/ou malades pouvant présenter des désordres du système cutané tels que notamment :
- les complications infectieuses telles que dermite atopique surinfectée, eczéma impétiginisé, ulcère, plaies, brûlures, acné inflammatoire surinfecté
- les polydermites telles que impétigo, folliculites superficielles,
- les dermites séborrhéiques, pityriasis versicolor
- les dermatophytoses (Tinea capitis, Tinea corporis, Pied d'athlète, Eczéma marginé de Hébra, Herpès circiné)
- les candidoses (vaginales, interdigitales, liées à des professions à risque ou au diabète)
- les désordres liés à des thérapeutiques avec des antibiotiques ou à des antimycosiques,
- les désordres entrainés par des dysrégulations hormonales (peaux grasses) ou à des états pelliculaires
- les peaux sensibles (prématurés, enfants).

Les compositions à usage vétérinaire sont particulièrement destinées à traiter ou prévenir les dysfonctionnements dus à des infections staphylococciques (à *Staphylococcus aureus, S. intermedians*), streptococciques *(à S. pyogenes*) et mycosiques (candidoses à *C. albicans* et pytirosporoses à *P. canis*).

D'autres caractéristiques de la présente invention apparaîtront au cours des descriptions suivantes d'exemples de formes de réalisation qui sont fournis à des fins d'illustration de la présente invention et qui ne sont pas destinés à être limitatifs.

### Exemples

### Exemple 1: Sélection de l'agent bactérien

Dans le cadre de la présente invention, on étudie l'adhésion de 12 souches différentes de bactéries à des cellules de peau, notamment à des kératinocytes humaines HaCat en culture. Ces souches appartiennent au genre des *Lactobacillus, Bifidobacterium, Micrococcus, Staphylococcus, Streptococcus, Propionibacterium.*

Les cultures de bactéries (1ml) sont incubées dans 10 ml de milieu (cf Table 1) pendant une nuit. Pour les essais d'adhésion, les bactéries sont précultivées jusqu'à une concentration de 5.0E+08 à 1.0E+09 cfu/ml. Les cfu sont standardisées par mesure de densité optique de chaque souche (DO à 1.0E+08 cfu/ml: voir Table 1).

Puis les souches de bactéries sont testées pour leur propriétés d'adhésion.

**Table 1:**

| **souches de bactéries et conditions de culture** | | | | |
|---|---|---|---|---|
| Souches de bactéries | code NCC | Milieu | Incubation T°C/heure | DO à 1E+02 cfu |
| *Lactobacillus johnsonii* La1 | 533 | MRS | Anaerob. 37°C /48h | 1.00 |
| *Lactobacillus acidophilus* La10 | 90 | MRS | Anaerob. 37°C /48h | 1.00 |
| *Bifidobacterium lactis* ATCC27536 | 536 | MRS | Anaerob. 37°C /18h | 0.65 |
| *Bifidobacterium longum* B28 | 585 | MRS | Aerob. 30°C /18h | 1.32 |
| *Micrococcus varians* NCC 1482 | 1482 | BHi | Aerob. 30°C/18h | 4.85 |
| *Micrococcus varians* NCC 1520 | 1520 | BHi | Aerob. 30°C/18h | 3.47 |
| *Micrococcus varians* MCV 17 | 1583 | BHi | Aerob. 30°C/18h | 4.18 |
| *Staphylococcus carnosus* STC 21 | 931 | BHi | Aerob. 30°C/18h | 40.20 |
| *Staphylococcus piscifermentans* STF4 | 751 | BHi | Aerob. 30°C/18h | 3.26 |
| *Streptococcus thermophilus* Sfi 16 | 2019 | HJL | Aerob. 40°C/18h | 0.37 |
| *Propionibacterium shermanii* PP12 | 1197 | MRS | Aerob. 30°C/24h | 0.18 |
| *Propionibacterium thoenii* PP22 | 1116 | MRS | Aerob. 30°C /24h | 0.25 |
| NCC 533, NCC 90, NCC536 et NCC585 sont été cultivées dans des conditions anaérobiques (Gaspack H2+ CO2). | | | | |

### Lignées de kératinocytes humains:

Les propriétés d'adhésion des bactéries ont été étudiées sur 3 lignées de kératinocytes :
- Lignées de cellules immortalisées SV40 T-Ag: cellules DK2-NR et FK2-NR telles que décrites dans EP 780 469 et,
- Lignées de cellules immortalisées HPV (Human papilloma virus) E6/E7 et SV40 T-Ag : lignées de cellules DK7-NR telles que décrites dans la demande WO 99/02347.

Milieu de culture pour les lignées de cellules:
DK7-NR, FK2-NR: NR-2 (Biofluids, Rockville, MD 20850) (EP 780469).
DK2-NR: NR-M (Biofluids, Rockville, MD 20850).

Les lignées de kératinocytes sont mises en culture à raison de 5x10⁵ keratinocytes/cm², ensemencés dans des clusters 6-puits coatés (Becton Dickinson, lincoln Park, NJ). La solution de coating est constituée de milieu basal supplémenté avec 10 µg/ml de fibronectine humaine (Becton Dickinson), 31µg/ml de collagène bovin I (Vitrogen, Collagen Corporation, Fremont, CA) et 0.1mg/ml de BSA (Biofluids, Rockville, MD 20850). Après 6 à 8 jours, les cultures de cellules forment une monocouche (confluent). Ensuite, la concentration du milieu en Ca²⁺ est amenée à 1.5mM pour induire la différenciation cellulaire. Les cellules sont mises en culture pendant 4 jours dans un milieu à haute concentration en Calcium, sans antibiotique. Pour les tests d'adhésion, les cultures de cellules sont lavées 3 fois avec du tampon (HBSS, Ca²⁺: 1.0mM).

### Radiomarquage

Les souches de bactéries sont marquées pendant une nuit par addition de 100 µCi/10 ml de 2-³H-adénine (Amersham, TRK.311) dans le milieu de culture. Des fractions aliquotes des bactéries sont incubées dans un milieu sans ³H-adénine. Le surnageant non marqué (froid) est mis de côté pour ajuster les cfu/ml pour les essais d'adhésion.

### Essais d'adhésion

Les suspensions de bactéries sont centrifugées pendant 10 minutes à 4000 tr/min. Avant l'ajustement de la densité optique (DO), les culots sont lavés 2 fois dans HBSS. La DO est mesurée pour chaque souche de manière à ajuster les concentrations finales en bactéries à 1.0E+06, 1.0E+07, 1.0E+08 et 1.0E+09 cfu/ml. Le milieu pour les essais d'adhésion est un mélange 1:1 de milieu de culture des kératinocytes et du surnageant non marqué du milieu bactérien.

Pour analyser les propriétés d'adhésion des bactéries sur un substrat sans kératinocytes, les suspensions de bactéries sont incubées sur des plats en plastique et des plats en plastiques coatés sans cellules.

### Analyse

Après lavage des cultures 3 fois avec de l'HBSS (Ca2+, 1,0mM) les bactéries associées aux kératinocytes sont lysées dans une solution de NaOH 1N pendant 30 minutes à température ambiante. La solution est transférée dans des fioles à scintillation avec 1 ml d'hydroxyde de benzethonium (Sigma, St. Louis, USA). Après 1 h à 60 °C, l'activité ³H des bactéries marquées est mesurée par comptage à scintillation liquide (dpm).

L'indice d'adhésion (AI) est calculé en activité ³H (dpm/puit) en % du total de l'activité ³H (dpm/ ml) de la suspension de bactéries.

### Résultats

### Indice d'adhésion (AI)

L'indice d'adhésion des 13 souches de bactéries différentes est calculé en mesurant l'activité ³H-adénine des micro-organismes radiomarqués. Les résultats sont présentés Table 2.

**Table 2:**

| **Indice d'adhésion de souches de bactéries sur kératinocytes FK2-NR.** | | | | |
|---|---|---|---|---|
| Code NCC | CFU/ml | dpm (x10³) | =SD | indice d'adhésion (% of total dpm) |
| 533 | 1.0E+09 | 209.6 | 24.2 | 1.2 |
| | 1.0E+08 | 175.5 | 42.5 | 10.4 |
| | 1.0E+07 | 39.5 | 1.9 | 23.3 |
| | 1.0E+06 | 4.7 | 0.3 | 27.7 |
| 90 | 1.0E+09 | 167.7 | 19.4 | 2.8 |
| | 1.0E+08 | 47.7 | 2.9 | 7.9 |
| | 1.0E+07 | 8.3 | 0.5 | 13.8 |
| | 1.0E+06 | 0.9 | 0.1 | 15.5 |
| 536 | 1.0E+09 | 413.9 | 91.7 | 4.8 |
| | 1.0E+08 | 221.0 | 31.3 | 25.5 |
| | 1.0E+07 | 22.3 | 3.8 | 25.8 |
| | 1.0E+06 | 2.4 | 0.3 | 28.2 |
| 585 | 1.0E+09 | 107.7 | 21.0 | 1.2 |
| | 1.0E+08 | 19.6 | 1.5 | 2.2 |
| | 1.0E+07 | 9.7 | 1.9 | 10.8 |
| | 1.0E+06 | 1.3 | 0.1 | 14.3 |
| 1482 | 1.0E+09 | 6147.2 | 1292.6 | 72.4 |
| | 1.0E+08 | 257.7 | 52.6 | 30.3 |
| | 1.0E+07 | 10.4 | 0.8 | 12.3 |
| | 1.0E+06 | 1.7 | 0.2 | 19.7 |
| 1520 | 1.0E+09 | 121.2 | 22.3 | 1.5 |
| | 1.0E+08 | 40.1 | 5.7 | 5.0 |
| | 1.0E+07 | 16.5 | 5.5 | 20.7 |
| | 1.0E+06 | 2.3 | 0.2 | 29.0 |
| 1583 | 1.0E+09 | 41.3 | 9.7 | 0.7 |
| | 1.0E+08 | 12.9 | 1.3 | 2.2 |
| | 1.0E+07 | 7.5 | 0.5 | 12.7 |
| | 1.0E+06 | 1.1 | 0.1 | 18.7 |
| 751 | 1.0E+09 | 195.5 | 96.5 | 2.6 |
| | 1.0E+08 | 24.7 | 4.9 | 3.2 |
| | 1.0E+07 | 4.8 | 0.9 | 6.4 |
| | 1.0E+06 | 0.6 | 0.09 | 8.9 |
| 931 | 1.0E+09 | 16.2 | 2.9 | 0.5 |
| | 1.0E+08 | 4.6 | 0.6 | 1.3 |
| | 1.0E+07 | 1.6 | 0.1 | 4.6 |
| | 1.0E+06 | 0.2 | 0.01 | 6.7 |
| 2019 | 1.0E+08 | 67.5 | 1.4 | 1.4 |
| | 1.0E+07 | 10.0 | 0.4 | 2.1 |
| | 1.0E+06 | 1.1 | 0.1 | 2.4 |
| 1197 | 1.0E+09 | 20.2 | 3.1 | 0.2 |
| | 1.0E+08 | 3.2 | 0.2 | 0.3 |
| | 1.0E+07 | 0.5 | 0.2 | 0.4 |
| | 1.0E+06 | 0.1 | 0.02 | 0.6 |
| 1116 | 1.0E+09 | 34.1 | 2.0 | 0.2 |
| | 1.0E+08 | 6.3 | 0.2 | 0.4 |
| | 1.0E+07 | 0.7 | 0.1 | 0.5 |
| | 1.OE+06 | 0.1 | 0.01 | 0.7 |

Les résultats montrent que les indices d'adhésion les plus forts sont obtenus pour les souche suivantes: une souche de *Bifidobacterium lactis* ATCC 27536, une souche de *Lactobacillus johnsonii* NCC 533 (CNCM I-1225) et les souche de *Micrococcus varians* NCC 1482 (CNCM I-1586), NCC 1520 (CNCM I-1587) et NCC 1583.

### Exemple 2: Tests in vitro de l'inhibition de l'adhésion de Staphylococcus aureus et Streptococcus pyogenes par Micrococcus varians NCC 1482 ou Lactobacillus johnsonii NCC 533.

### Microorganismes et méthodes de culture

Les pathogènes *Staphylococcus aureus, Streptococcus pyogenes* sont cultivés en bouillon par repiquage à partir d'une culture sur pente (Table 3 ). Une correspondance DO/densité bactérienne a été établie pour chacun des germes testés, sur la base de dilutions sériées et de dénombrements sur milieu gélosé.

**Table 3**

| **Souche bactérienne** | **Ref.** | **milieu** | **Conditions de culture** |
|---|---|---|---|
| *Staphylococcus aureus* | ATCC 6538 | TCS | Aérobiose, 35°C / 24 h |
| *Streptococcus pyogenes* | CIP 5641 T | BHI | Aérobiose, 35°C / 24 h |

Milieux de culture:
TCS (AES, Combourg, ref. AEB 141502)
BHI (UNIPATH SA, Dardilly, ref. CM 225)

### Kératinocytes humains transformés

On utilise des kératinocytes humains immortalisés de la lignée HaCaT (Boukamp P. et coll., *J. Cell Biol.,* 106, 761-771, 1988). Les cellules HaCaT sont cultivées en milieu DMEM supplémenté par 10% de serum de veau foetal, à 37°C sous 5% de CO₂.

Des clusters 6 puits (Becton Dickinson) sont ensemencés à raison de 10⁴ cellules/cm². Après 4 à 5 jours les cellules atteignent la confluence. Les essais d'adhésion sont réalisés 4 à 5 jours après la confluence. Les monocouches sont lavées 3 fois au PBS avant ces essais.

### Radiomarquage

Les bactéries sont marquées à la 2-³H-adénine (Amersham, TRK 311), à raison de 100 µCi/10 ml de bouillon. Les suspensions sont lavées 3 fois puis resuspendues en PBS. La densité cellulaire est ajustée dans ce même tampon.

### Essai d'adhésion sur kératinocytes en culture:

1ml de suspension bactérienne radiomarquée est incubée pendant 1h à 35°C. La monocouche est lavée 3 fois par du tampon PBS et lysées par addition de NaOH 1N pendant 30 minutes à température ambiante. Le lysat est transféré en fiole à scintillation et incubé 1h à 60°C avec 1ml d'hyamine hydroxide (Carlo Erba, ref. 464951). L'activité ³H est comptée au compteur à scintillation liquide. Chaque essai est réalisé en triple. Un contrôle au support plastique est également réalisé.

L'adhésion est définie par le rapport entre radioactivité adhérée et radioactivité introduite, multiplié par 100.

### Essai d'inhibition d'adhésion

Après lavage et resuspension en PBS, le pathogène radiomarqué et la souche de bactérie froide sont incubés simultanément avec la monocouche. Les essais sont effectués en triple pour des densités en agent bactérien couvrant 3 log.

### Résultats

*Staphylococcus aureus, Streptococcus pyogenes, Lactobacillus johnsonii* NCC 533 et *Micrococcus varians* NCC 1482 ont été testées pour leur adhésion aux kératinocytes HaCaTen culture. Les résultats sont donnés dans la Table 4.

**Table 4**

| | **Adhésion (%)** | | |
|---|---|---|---|
| **Micro organisme** | **1.0E+06 cfu/ml** | **1.0E+07 cfu/ml** | **1.0E+08 cfu/ml** |
| *Staphylococcus aureus* | - | 5,0 | 2,5 |
| *Streptococcus pyogenes* | - | 35 | 42,5 |
| NCC 533 | 4,5 | 1,7 | 1,2 |
| NCC 1482 | 6,5 | 3,0 | 0,6 |

| | **Inhibition (%)** | | |
|---|---|---|---|
| | **9.0E+06 cfu/ml** | **9.0E+07 cfu/ml** | **9.0E+08 cfu/ml** |
| *S. aureus* 1E+8 cfa/ml + NCC 1482 | 5 | 11 | **66** |
| *S. aureus* 1E*+*8 cfu/ml + NCC 533 | 20 | 24 | 34 |
| *S. pyogenes* 1E+8 cfu/ml+ NCC 1482 | 26 | 28 | 40 |
| *S.pyogenes* 1E+8 cfu/ml+NCC533 | 12 | 19 | **52** |

Les résultats montrent que *Staphylococcus aureus, Streptococcus pyogenes*, *Lactobacillus johnsonii* NCC 533 et *Micrococcus varians* NCC 1482 adhèrent aux kératinocytes en culture. Lorsque la densité de l'agent bactérien augmente, le pathogène est de plus en plus déplacé.

### Exemple 3: Tests in vitro de l'inhibition de l'adhésion de S.aureus par Lactobacillus johnsonii NCC 533 actif ou désactivé.

Le modèle d'adhésion in vitro repose sur l'incubation d'une suspension radiomarquée et calibrée d'un microorganisme pathogène cutané *(Staphylococcus aureus*) avec une monocouche de kératinocytes humains immortalisés (lignée HaCaT (Boukamp P. et coll., *J. Cell Biol.,* 106, 761-771, 1988).

L'activité inhibitrice de l'agent bactérien *(Lactobacillus johnsonii* NCC 533 sous forme viable ou désactivée) vis-à-vis de cette adhésion est évaluée dans le cadre d'une co-incubation sur la monocouche du pathogène et du composé à tester par dosage de la radio-activité retenue sur la monocouche.

### Kératinocytes

Pour cela, les cellules HaCaT sont cultivées en milieu DMEM supplémenté par 10% de serum de veau foetal, à 37°C sous 5% de CO₂. Elles sont ensemencées en cluster 6 puits au taux de 1.0E+04 cellules/cm². L'essai d'adhésion est réalisé 5 jours après confluence. Les monocouches sont lavées 3 fois au PBS avant incubation avec les microorganismes.

### Microorganismes

*Staphylococcus aureus* (ATCC 6538) est cultivé en milieu TCS en aérobiose à 35°C.

Lactobacillus *johnsonii* NCC 533 est cultivé en milieu MRS en anaérobiose à 37°C. Pour l'essai d'inhibition d'adhésion, une suspension concentrée de la bactérie est préparée en tampon PBS à partir d'une culture de 48 heures. La suspension est ajustée à 2x10⁸ cfu/ml (DO à 525nm = 1,5). Des dilutions sérielles sont réalisées en tampon PBS afin d'obtenir des suspensions à 2.0E+07 et 2.0E+06 cfu/ml. Les différentes suspensions sont dénombrées sur gélose MRS incubée en anaérobiose à 37°C.

La forme désactivée de NCC 533 est obtenue par lyophilisation d'une suspension dense de Lactobacilles soumise à plusieurs cycles de congélation en azote liquide / décongélation à température ambiante. La préparation testée correspond à une biomasse de 4.0E+10 cfu/g.

### Radiomarquage

Le radiomarquage de *Staphylococcus aureus* est obtenu par incorporation de 100 µCi/10 ml de ³H adénine pendant 24h de culture en bouillon TCS. La suspension est alors centrifugée 10 minutes à 3000 tr/min et lavée 3 fois en PBS. La densité cellulaire est ajustée avec du tampon PBS à environ 2.0E+08 cfu/ml (DO à 525nm=0,5). La radioactivité spécifique est déterminée par comptage en scintillation sur 100 µl de la suspension.

### Essai d'inhibition d'adhésion:

On ajoute simultanément 1ml de suspension radiomarquée de *Staphylococcus aureus* et 1ml de suspension de NCC 533 par puits de culture de cellules HaCaT. Après 1h d'incubation à 37°C, les monocouches sont lavées 3 fois par du tampon PBS et lysées par addition de 1ml de NaOH 1N pendant 30 minutes à température ambiante. Le lysat est transféré en fiole à scintillation et incubé 1h à 60°C avec 1ml d'hydroxyde de benzéthonium. Après refroidissement, 10 ml de liquide de scintillation Hyonic fluor sont ajoutés et la radioactivité est comptée au compteur à scintillation liquide. Le contrôle est obtenu par ajout de 1ml de suspension radiomarquée de *Staphylococcus aureus* et lml de tampon PBS et correspond au 100% d'adhésion.

Les résultats sont donnés dans la Table 5.

**Table 5:**

| **Inhibition de l'adhésion de *Staphylococcus aureus* aux cellules HaCaT par NCC 533 sous sa forme viable et sous sa forme désactivée.** | | |
|---|---|---|
| | Adhésion (%) | Ecart-type |
| Contrôle | 100 | 9 |
| NCC 533 3.0E+06 cfu/ml | 74 | 19 |
| NCC 533 3.0E+07 cfu/ml | 69 | 8 |
| NCC 533 3.0E+08 cfu/ml | 34 | 1 |
| NCC 533 désactivé 3.0E+06 cfu/ml | 27 | 2 |
| NCC 533 désactive 3.0E+07 cfu/ml | 12 | 1 |
| NCC 533 désactive 3.0E+08 cfu/ml | 6 | 0 |

### Exemple 4: Tests in vivo de l'inhibition de l'adhésion de pathogènes cutanés par Lactobacillus johnsonii désactivé.

### Materiel et méthodes

### Animaux:

15 souris femelles SKH âgées de 7 à 8 semaines et pesant environ 30g ont été fournies par C. River. On a utilisé 5 souris pour chaque groupe testant une application topique différente.

### Microorganisme

On utilise une souche de *Staphylococcus aureus* (appelée : souche 1) qui a été isolée à partir d'une lésion de peau humaine (ulcère de la jambe). Cette souche est sensible à la Methyciline.

### Préparation de l'inoculum

On prépare une suspension de la bactérie pour son inoculation aux souris. Pour cela, une préculture en pente de la souche 1 est effectuée sur un milieu solide (AES, AEB 122 859) à 35°C pendant 18 à 24h. Après l'incubation, la bactérie est resuspendue dans 10 ml de solution saline stérile, puis récupérée après centrifugation à 3000 pendant 10 min. Le surnageant est ensuite éliminé et le culot est repris par 10 ml de solution saline. Cette procédure est répétée 2 fois. Une suspension inoculum est preparée en resuspendant les bactéries lavées dans 4 ml de solution saline stérile. La DO à 525 nm est ajustée à environ 0,14. Elle contient environ 10⁸ cfu/ml.

### Inoculation des souris

La peau des souris est délipidée au niveau des flancs avec de l'éthanol 95 (Merck). 50 µl d'une suspension contenant un mélange 50/50 de l'inoculum de *S. aureus* 1.0E+07 cfu/ml et du produit à tester ont été lentement appliqués à l'aide d'une micropipette sur la zone délipidée (6,25 cm2). Les sites inoculés sont protégés par occlusion pendant 1 h sous un pansement plastique stérile (Dermafilm 33x15, ref 38.3015, Vygon laboratory).

### Comptage des bacteries viables des lésions

4 heures après l'application de la suspension, les souris sont tuées sous anesthésie avec du forene (Abbott France). Les sites inoculés sont excisés en bloc (diamètre 12 mm). Les biopsies de peaux prélevés sont broyés et homogénéisés avec 2 ml de solution saline stérile avec un Polytron (PT 2100, Bioblock Scientific) (5tr/min, 5 min.).

Un échantillon de 1ml du tissu homogénéisé est ajouté à 9 ml d'une solution saline stérile et 0,1ml de ce mélange est cultivé sur un milieu staphyloccique n°110 en utilisant la méthode dilutron 10 fois. Après 48 heures d'incubation, à 35°C, les colonies développées sont comptées et les CFU(unités de formation de colonies) sont déterminées.

### Résultats

Les résultats sont présentés en Table 6.

**Table 6:**

| **essai du NCC 533 à 1%** | |
|---|---|
| | Log cfu/cm2 |
| *S.auréus* + PBS | 3,4 |
| *S.auréus* + NCC 533 1 % dans PBS | 2,6* |
| *S.auréus* + Glutaraldéhyde 0,045% | 0,8* |

| | |
|---|---|
| * significatif par rapport au témoin (SA/PBS), test T de Student (n=5) | |

La présence de NCC 533 à 1% permet de réduire le nombre de bactéries retrouvées d'environ 1 log après 4 heures de contact. La différence apparaît significative par rapport au témoin (p=0,098).

En présence de glutaraldéhyde, la diminution du nombre de bactéries est encore plus importante par rapport au témoin, cependant il n'est pas exclu que cette activité soit due à son activité antiseptique et agisse dès la mise en présence du S.aureus dans le mélange; ainsi l'activité observée après 4 h ne serait pas du à un effet anti-adhésif mais à une activité anti-bactérienne du produit.

Les résultats sont représentés en Table 7.

**Table 7**

| Taille d'inoculum cfu/ml | Traitement | % de réduction vs. témoin | Significativité vs. témoin |
|---|---|---|---|
| | NCC 533 0,5% | 20,1 % | P= 0,019 (*) |
| 1.0E+06 cfu/ml | NCC 533 1% | 22,6 % | P= 0.016 (*) |
| | Glutaraldéhyde 0,045% | 87,9 % | P= 0,0001 (***) |
| | NCC 533 0,5% | 27,4 % | P= 0,0015 (*) |
| 1.0E+07 cfu/ml | NCC 533 1% | 19,9 % | P= 0.0004 (***) |
| | Glutaraldéhyde 0,045% | 92,0 % | P= 0,0004 (***) |
| * p<0.05, *** p<0.001 significatif par rapport au témoin (SA/PBS), | | | |

La présence de NCC 533 à 0,5 % et 1 % réduit le nombre de *S.aureus* retrouvés d'environ 1 log pour les inocula à 10⁶ cfu/ml et 10⁷ cfu/ml. On n'observe pas d'effet dose, que ce soit avec l'inoculum 1.0E+06 cfu/ml ou avec l'inoculum à 1.0E+07 cfu/ml.

En présence de glutaraldéhyde à 0,045%, la diminution est beaucoup plus importante par rapport au témoin, elle est d'environ 3 log.

Ces résultats confirment l'activité du NCC 533 désactivé à 0,5% et à 1% comme inhibiteur de l'adhésion de *S.aureus.*

### Exemple 5 : lotion pour le corps

On prépare une lotion pour le corps ayant la composition suivante : 8,0% d'huile minérale, 5,0% d'isopropyl palmitate, 2,0% de polyglycéryl-3-diisostéarate, 4,0% d'Octyldodecanol, 0,3% de carbomère, 0,2% de sodium cocoylglutamate, 1,2%d' hydroxyde de sodium à 10%, un conservateur, du parfum, 0,5 à 3% d'un lyophylisat contenant de 10E+8 à 10E+12 cfu/g d'au moins une souche de bactérie choisie parmi les *Lactobacillus johnsonii* (CNCM I-1225), *Micrococcus varians* (CNCM I-1586 ou CNCM I-1587) ou *Bifidobactérium lactis* (ATCC 27536, Hansen) et inactivée par traitement thermique à environ 90°C pendant environ 2 heures. On complète à 100% avec de l'eau.

La lotion pour le corps ainsi obtenue grâce à ses propriétés d'anti-adhésion vis-à-vis des pathogènes, est destinée à stabiliser et/ou réguler la flore pathogène de la peau.

### Exemple 6 : shampooing

On prépare un shampooing ayant la composition suivante : 7,0% de sodium lauryl sulphate, 2,0% de cocamidopropylbetain, 2,0% de sodium lauryl sulphonosuccinate, chlorure de sodium, conservateur, parfum et de 0,5 à 3% d'un lyophylisat contenant de 1.0E+08 à 1.0E+12 cfu/g d'au moins une souche de bactérie choisie parmi les *Lactobacillus johnsonii* (CNCM I-1225), *Micrococcus varians* (CNCM I-1586 ou CNCM I-1587) ou *Bifidobactérium lactis* ATCC 27536, et inactivée par traitement thermique à environ 90°C pendant environ 2 heures. On complète à 100% avec de l'eau.

Le shampoing ainsi préparé à des propriétés régulatrices de la flore pathogène du cuir chevelu. Il est notamment indiqué dans le traitement des états pelliculaires.

### Exemple 7

Pour obtenir une composition pharmaceutique ayant des propriétés régulatrices de la flore pathogène cutanée, on prépare les phases grasses et aqueuses ayant la composition suivante :

| | | |
|---|---|---|
| | *L. johnsonii* (CNCM I-1225) tel que décrit à l'exemple 5 | 1% |
| Phase grasse: | Arachidyl behenyl alcohol/arachidylglucoside | 3% |
| | Isohexadecane | 7% |
| | Huile d'amande douce | 3% |
| | Beurre de Karité | 2% |
| | B.H.T. | 0,05% |
| | POB Propyle | 0,05% |
| Phase aqueuse: | Eau | Qs 100% |
| | Glycérine | 5% |
| | POB Methyle | 0,1% |

On chauffe les phases grasses et aqueuse à 75°C. Puis on émulsifie par addition de la phase aqueuse dans la phase grasse sous une agitation Rayneri de 1000 Tr/min. 30 minutes après l'émulsification, on homogénéise 1 minute au polytron (vitesse 4-5).

### Exemple 8

On prépare une composition de la même manière qu'à l'exemple 7, mais ayant la composition suivante:

| | | |
|---|---|---|
| | *L. johnsonii* (CNCM I-1225) tel que décrit à l'exemple 5 | 1% |
| Phase grasse: | Glyceryl Stearate et PEG100 Stearate | 5% |
| | Isohexadecane | 8% |
| | Beurre de Karité | 5% |
| | B.H.T. | 0,05% |
| | Dc 1503 | 1% |
| Phase aqueuse: | Eau | Qs 100% |
| | Glycérine | 3% |
| | Carbopol 981 | 0,2% |
| | Lubrajel | 5% |
| | Phenoxyethanol | 1% |
| | Soude | Qs pH6 |

### Exemple 9

On prépare une composition de la même manière qu'à l'exemple 7, mais ayant la composition suivante :

| | | |
|---|---|---|
| | *L. johnsonii* (CNCM I-1225) tel que décrit à l'exemple 5 | 1 % |
| Phase grasse: | Polyglyceryl-3-Diisostearate | 5% |
| | Cyclomethicone CM5 | 20% |
| Phase aqueuse: | Eau | Qs 100% |
| | Glycérine | 5% |
| | NaCl | 0,5% |
| | MgSO4 | 0,5% |

## Revendications

1. Utilisation d'un agent bactérien pour la préparation de compositions à usage cosmétique, pharmaceutique ou vétérinaire, destinées à être administrées à l'homme ou à des animaux dans un but thérapeutique ou prophylactique de stabilisation et/ou de régulation de la flore pathogène du système cutané, ledit agent bactérien étant un extrait de bactérie ou une bactérie sélectionnée pour ses facultés d'adhésion vis-à-vis des cellules de la peau et ses propriétés anti-adhésives vis-à-vis des pathogènes du système cutané.

2. Utilisation selon la revendication 1, dans laquelle l'agent bactérien est choisi parmi les espèces de *Lactobacillus, Micrococcus* ou *Bifidobacterium.*

3. Utilisation selon l'une des revendications 1 ou 2, dans laquelle l'agent bactérien est choisi parmi les souches de *Lactobacillus johnsonii* CNCM I-1225, *Micrococcus varians* CNCM I-1586, *Micrococcus varians* CNCM I-1587 ou *Bifidobactérium lactis* ATCC 27536.

4. Utilisation selon l'une des revendications 1 à 3, dans laquelle la bactérie est sous forme viable, semi-active ou désactivée.

5. Utilisation selon la revendication 4, dans laquelle la bactérie est désactivée par traitement thermique à 90°C pendant 2 heures.

6. Utilisation selon l'une des revendications 1 à 5, caractérisée en ce que l'agent bactérien sous forme lyophilisée contient de 1.0E+08 à 1.0E+11 cfu/g.

7. Composition à usage cosmétique, phamaceutique ou vétérinaire contenant au moins un agent bactérien capable de stabiliser et/ou de réguler la flore pathogène du système cutané, ledit agent bactérien étant un extrait de bactérie ou une bactérie sélectionnée pour ses facultés d'adhésion vis-à-vis des cellules de la peau et ses propriétés anti-adhésives vis-à-vis des pathogènes du système cutané.

8. Composition selon la revendication 7, dans laquelle l'agent bactérien est choisi parmi les espèces de *Lactobacillus, Micrococcus* ou *Bifidobactérium.*

9. Composition selon l'une des revendications 7 ou 8, dans laquelle l'agent bactérien est choisi parmi les souches de *Lactobacillus johnsonii* CNCM I-1225, *Micrococcus varians* CNCM I-1586, *Micrococcus varians* CNCM I-1587 ou *Bifidobactérium lactis* ATCC 27536.

10. Composition selon l'une des revendications 7 à 9, dans laquelle la bactérie est sous forme viable, semi-active ou désactivée.

11. Composition selon la revendication 10, dans laquelle la bactérie est désactivée par traitement thermique à 90°C pendant 2 heures.

12. Composition selon l'une des revendications 7 à 11, contenant jusqu'à 10% en poids de l'agent bactérien par rapport au poids total de la composition.

13. Composition selon l'une des revendications 7 à 12, contenant environ 0,05 à 5% en poids de l'agent bactérien par rapport au poids total de la composition.

14. Composition selon l'une des revendications 7 à 13, destinée au traitement ou à la prophylaxie des désordres cutanés tels que :
- les complications infectieuses telles que dermite atopique surinfectée, eczéma impétiginisé, ulcère, plaies, brûlures, acné inflammatoire surinfecté
- les polydermites telles que impétigo, folliculites superficielles,
- les dermites séborrhéiques, pityriasis versicolor
- les dermatophytoses
- les candidoses
- ceux liés à des thérapeutiques avec des antibiotiques ou à des anti-mycosiques,
- ceux entrainés par des dysrégulations hormonales ou à des états pelliculaires

15. Composition selon l'une des revendications 7 à 14 destinée à être appliquées sur les peaux sensibles ou les peaux grasses.

16. Composition à usage vétérinaire selon l'une des revendications 7 à 13, destinée au traitement ou à la prévention des dysfonctionnements liés à des infections staphylococciques, streptococciques et mycosiques chez les animaux.
